# FASCICULE DE BREVET EUROPEEN

(11) **EP 1 123 089 B1**
(45) Date de publication et mention de la délivrance du brevet: **10.09.2003**
(21) Numéro de dépôt: 99950805.4
(22) Date de dépôt: 21.10.1999
(51) Int. Cl.: A61K 9/50, A61K 9/20

(54) **COMPRIME A CROQUER A GOUT MASQUE ET LIBERATION IMMEDIATE DU PRINCIPE ACTIF ET PROCEDE DE FABRICATION**
GESCHMACKSMASKIERTE KAUTABLETTE MIT SOFORTIGER WIRKSTOFFFREISETZUNG SOWIE HERSTELLUNGSVERFAHREN
TABLET FOR CRUNCHING WITH MASKED TASTE AND INSTANT RELEASE OF ACTIVE PRINCIPLE AND METHOD FOR MAKING SAME

(30) Priorité: 23.10.1998 FR 9813466
(43) Date de publication de la demande: 16.08.2001
(73) Titulaire: GATTEFOSSE S.A., 69800 Saint Priest (FR)
(72) Inventeur: BARTHELEMY, Philippe, F-69780 Mions (FR); BENAMEUR, Hassan, F-69740 Genas-Azieu (FR); CRUMINIAN, Géraldine, F-26200 Montelimar (FR)
(74) Mandataire: Vuillermoz, Bruno
(86) Numéro de dépôt international: FR9902563
(87) Numéro de publication internationale: WO00024385

(56) Documents cités:
- EP-A- 0 322 048
- EP-A- 0 408 273
- EP-A- 0 839 526
- WO-A-94/12180
- WO-A-98/14176
- US-A- 5 891 476
- DATABASE WPI Week 9721 Derwent Publications Ltd., London, GB; AN 97-231144 XP002107162 & JP 09 071523 A (RYUKAKUSAN KK), 18 mars 1997 (1997-03-18)

## Description

L'invention se rapporte à un comprimé à croquer à goût masqué et à libération immédiate du principe actif. Elle concerne également le procédé de fabrication dudit comprimé.

Certains principes actifs ne peuvent être incorporés dans des formes galéniques du type comprimés à croquer du fait de leur amertume élevée ou de leur mauvais goût. C'est le cas par exemple, mais de façon non limitative de l'ibuprofène, de l'acide acétylsalicylique, du paracétamol, de la cimétidine, etc.

Pour réduire l'amertume de ces principes actifs, on a proposé, notamment dans le document EP-A-459 695 des comprimés à croquer constitués de granules de principe actif enrobés d'acétate de cellulose et/ou de butyrate acétate de cellulose en mélange avec de l'hydroxypropyl cellulose dans un solvant organique.

On a également proposé dans le document US-A-4 851 221 des comprimés à croquer d'acétaminophène préparés à partir de granulés enrobés de polyvinyle pyrrolidone (PVP) toujours solubilisé dans un solvant organique tel que l'acétone et/ou le méthanol.

Dans les deux cas, les grains ainsi obtenus sont ensuite compressés avec un mélange d'excipients usuels pour la fabrication de comprimés à croquer, tels que agent diluant, agent liant, agent désintégrant, agent lubrifiant, agent édulcorant et éventuellement agent colorant.

Toutefois, les comprimés à croquer décrits dans ces documents présentent un certain nombre d'inconvénients.

On note tout d'abord le nombre important de constituants nécessaires à la réalisation de l'enrobage des grains de principe actif et notamment la présence indispensable d'un solvant organique. Or, les contraintes réglementaires obligent à recouvrer les solvants organiques dans le but de protéger l'environnement, nécessitant des investissements onéreux. De même, les autorités réglementaires exigent de plus en plus l'absence de solvants organiques dans les préparations pharmaceutiques et à tout le moins le dosage de leurs traces. Or, ces dosages demandent du matériel sophistiqué et onéreux en raison des quantités infinitésimales de solvant à rechercher.

On souligne également la proportion importante d'enrobage qui est de l'ordre de 12 % en poids du poids de principe actif, ce qui augmente le coût de la préparation.

Enfin et surtout, les comprimés obtenus peuvent présenter un profil de libération prolongée de principe actif incompatible lorsque l'on souhaite une libération immédiate.

On a proposé dans le document WO 94/12180 des comprimés à croquer de cimétidine constitués de granules enrobés d'au moins 20% en poids par rapport à la cimétidine d'une huile végétale partiellement hydrogénée, par exemple du type huile de coton. Les granulé enrobés sont ensuite compressés avec un mélange d'excipients appropriés tels que agent diluant (lactose, xylitol..), agent désintégrant du type polyvinyl pyrrolidone réticulée ou carboxyméthyl cellulose réticulée....

Toutefois, de même que précédemment, les quantités d'enrobage sont importantes augmentant ainsi le coût de production et le temps de libération du principe actif. Par ailleurs, en utilisant des huiles naturelles, les résultats obtenus d'un lot à l'autre ne sont pas toujours reproductibles. Enfin, l'huile partiellement hydrogénée constituant l'enrobage présentant un caractère hydrophobe, elle ne peut adhérer convenablement sur le principe actif lorsque celui-ci présente un caractère hydrophile, de sorte que d'une part, le masquage du goût n'est pas satisfaisant et d'autre part, la libération du principe actif est irrégulière.

Le document WO 98/14176 du Demandeur concerne un procédé de fabrication de particules de principe actif enrobées d'un agent matriciel lipidique. Non seulement, ce document ne concerne pas des comprimés à croquer, mais au surplus l'exemple 3 indique clairement qu'alors que des particules de principe actif enrobées présentent un profil de libération immédiate, les mêmes particules compressées présentent au contraire un profil de libération prolongée.

Le document EP-A-0 322 048 décrit des comprimés à croquer de cimétidine obtenus par un procédé selon lequel on granule sous vide et à chaud un mélange de poudre constitué de cimétidine, d'ester de polyhydroxyalcool et d'agent émulsifiant, parallèlement on granule par voie humide un mélange de poudre constitué d'agent anti-acide et d'agent désintégrant du type carboxyl méthyl cellulose, les deux populations de granules étant ensuite compressées en présence d'adjuvants de compression usuels permettant d'obtenir des comprimés.

Selon ce procédé, les particules de principe actif sont agglomérées grâce à la présence d'ester de polyhydroxyalcool et d'agent émulsifiant conduisant ainsi à la formation de granules. De par ce mode de fabrication, nécessitant par ailleurs de fortes proportions d'ester (plus de 50 % dans le cas du Précirol ®), les granules obtenus présentent un profil de libération lent du principe actif (90 % dans un délai supérieur à 60 minutes).

Au regard de l'état de la technique précité, l'invention vise à résoudre simultanément cinq problèmes, respectivement :
◆ du point de vue de la composition même du principe actif enrobé :
   - réduire le nombre de constituants ;
   - réduire la proportion de l'enrobage et partant réduire le coût de production ;
◆ du point de vue de la libération du principe actif:
   - obtenir une libération immédiate de principe actif, c'est-à-dire une libération d'au moins 80 % du principe actif en moins de 30 minutes (correspondant à la norme USP XXIII);
◆ du point de vue des qualités organoleptiques ;
   - masquer le goût amer du principe actif ;
   - améliorer le caractère croquant du comprimé final.

Pour résoudre l'ensemble de ces problèmes, l'invention propose un comprimé à croquer à goût masqué et à libération immédiate du principe actif.

Ce comprimé se caractérise en ce qu'il est constitué d'un mélange comprenant:
- d'une part, des particules individualisées de principe actif enrobées d'un agent lipidique obtenu par réaction entre au moins un polyhydroxyalcool et au moins un acide gras saturé libre ou estérifié, ledit acide gras comprenant de 8 à 22 atomes de carbone ;
- et d'autre part, au moins un agent liant-désintégrant.

Dans la suite de la description et dans les revendications, par le terme "polyhydroxyalcool", on désigne une chaîne alkyl comprenant au moins deux fonctions alcool, et notamment mais de façon non limitative, le glycérol, le polyéthylène glycol, les sucres tels que le saccharose.

Les réactions entre polyhydroxyalcools et acides gras libres ou estérifiés sont parfaitement connus de l'homme du métier et sont plus particulièrement décrites dans le document BAILEYS INDUSTRIAL OILS AND FAT PRODUCTS édité par Daniel SWERN comme correspondant à des réactions d'estérification (polyhydroxyalcool + acide gras libre) ou d'interestérification parmi lesquelles l'alcoolyse (esters d'acides gras + alcool) ou encore la glycérolyse (esters d'acides gras + glycérol).

En d'autres termes, l'invention vise à proposer un comprimé à croquer dont les particules de principe actif ne sont pas enrobés de polymère filmogène en solution dans un solvant organique, mais d'un agent lipidique spécifique fondu et ce, en l'absence de tout solvant, conduisant donc à réduire le nombre de constituants nécessaires à l'enrobage.

En outre, l'agent lipidique sélectionné est un produit de synthèse issu de matière première naturelle, présentant donc un point de fusion et des caractéristiques physico-chimiques déterminés et donc reproductibles d'un lot à l'autre. De même, cet agent lipidique, tout en présentant les caractéristiques d'un lipide et notamment la caractéristique filmogène constituant barrière au mouvement hydrique, présente également un caractère amphiphile dû à la présence des groupements hydroxyle du polyhydroxyalcool non substitués. Il s'ensuit que l'agent lipidique présente une adhésivité améliorée aussi bien sur les supports hydrophiles que sur les supports hydrophobes permettant non seulement un masquage de goût efficace, mais également une libération régulière du principe actif, sans effet d'action prolongée.

Par ailleurs, les particules de principe actif sont enrobées individuellement d'agent lipidique, et ce sans agglomération aucune avec d'autres excipients permettant ainsi d'obtenir un film de faible épaisseur et homogène et par conséquent sans aucun effet d'action prolongée.

Enfin, la libération immédiate du principe actif résulte également de la combinaison entre l'enrobage des particules de principe actif et l'agent liant-désintégrant incorporé dans un mélange d'excipients usuels pour la fabrication de comprimés à croquer.

Dans la suite de la description et dans les revendications, par le terme "agent liant-désintégrant", on désigne un excipient apte à favoriser, d'une part, de par son caractère liant, la compression des poudres mélangées lors de l'étape de fabrication du comprimé, et d'autre part, de par son caractère désintégrant, la désagrégation du comprimé lorsque celui-ci est mis au contact d'un milieu liquide aqueux.

En d'autres termes, l'agent liant-désintégrant, de par son caractère liant, renforce la cohésion des poudres constituées des excipients de la formulation tout en permettant, de par son caractère désintégrant, la désintégration rapide des comprimés et partant, la libération , puis la dissolution du principe actif.

Afin d'améliorer la cohésion de l'enrobage lipidique sur les particules de rincipe actif, sans toutefois altérer la stabilité du comprimé, la température de fusion de l'agent lipidique est comprise entre 37 et 75°C.

Pour une température de fusion de l'agent lipidique inférieure à 37° C, la stabilité du film lipidique enrobant les particules de principe actif du comprimé peut être modifiée notamment par la chaleur, lors du stockage, ce qui n'est pas souhaitable.

En revanche, pour une température de fusion supérieure à 75° C, ladite température de fusion est trop éloignée de la température de 37° C de sorte que la dissolution du principe actif peut être prolongée, ce qui n'est pas recherché.

Avantageusement, l'agent lipidique est un ester de glycérol et d'un mélange d'acide stéarique et palmitique commercialisé par le Demandeur sous la marque PRECIROL® ATO 5 et correspondant à la monographie de la pharmacopée européenne 2000.1428 et listée dans le registre américain 21 CFR comme GRAS (Generally Recognized As Safe).

Comme déjà dit, le comprimé de l'invention grâce à sa composition permet de réduire considérablement la proportion d'enrobage, et donc de favoriser la libération rapide du principe actif en conjonction avec la désagrégation due à la présence de l'agent liant-désintégrant.

En effet, et en pratique, l'agent lipidique représente au plus 10 % en poids du poids du principe actif.

Pour un enrobage supérieur à 10%, on observe un effet prolongé de la libération du principe actif, suivant le test in vitro de dissolution prescrit par la pharmacopée américaine USP XXIII.

Avantageusement, l'agent lipidique représente de 2 à 5 % du poids de principe actif.

Selon une autre caractéristique de l'invention, l'agent liant-désintégrant est choisi dans le groupe comprenant l'hydroxypropyl éther de cellulose faiblement substitué.

Par hydroxypropyl éther de cellulose faiblement substitué, on désigne un hydroxypropyl éther de cellulose dans lequel une faible proportion des trois groupes hydroxyle contenus dans la chaîne β-o-glucopyranosyl de la cellulose sont estérifiés par de l'oxyde de propylène.

En pratique, l'hydroxypropyl éther de cellulose faiblement substitué contient de 7 à 16 % d'unité hydroxypropoxyl.

Avantageusement, l'hydroxypropyl éther de cellulose faiblement substitué contient de 7 à 12,9% d'unité hydroxypropoxyl correspondant à l'hydroxypropyl éther de cellulose faiblement substitué dénommé "LH 21" commercialisé par SHINETSU.

De même et selon une autre caractéristique de l'invention, l'agent liant-désintégrant représente entre 0,1 et 5 % en poids du poids d'un comprimé.

L'invention se rapporte également au procédé de fabrication de comprimés à croquer à goût masqué et libération immédiate de principe actif.

Ledit procédé comprend les étapes suivantes respectivement:
- une étape d'enrobage lors de laquelle :
   · tout d'abord, on chauffe tout en fluidisant, une poudre constituée de particules individualisées de principe actif à une température voisine du point de fusion de l'agent lipidique mis en oeuvre ultérieurement ;
   · on pulvérise ensuite, sur les particules individualisées en l'absence de tout solvant, toujours à chaud, à l'état fondu, un agent lipidique obtenu par réaction entre au moins un polyhydroxyalcool et au moins un acide gras saturé libre ou estérifié, ledit acide gras comprenant de 8 à 22 atomes de carbone ;
   · on refroidit les particules enrobées jusqu'à température ambiante;
- une étape de mise en forme lors de laquelle :
   · on mélange préalablement à température ambiante, les particules ainsi enrobées avec au moins un agent liant-désintégrant ;
   · puis, on soumet le mélange obtenu à l'étape de mise en forme proprement dite afin d'obtenir des comprimés.

Selon un premier mode de réalisation de l'invention, l'étape de mise en forme consiste en une compression.

Selon un second mode de réalisation de l'invention, l'étape de mise en forme consiste en une extrusion.

En d'autres termes, le procédé de l'invention consiste à mettre en oeuvre la technique dite de "hot melt coating" consistant à enrober à chaud la matière lipidique sur des particules de principe actif et ce, avant une étape ultérieure de compression ou extrusion du mélange constitué des particules enrobées et d'excipient, d'une part, spécifique tel que l'agent liant-désintégrant, et d'autre part, usuel de formulation de comprimés à croquer.

Bien entendu, on peut prévoir de granuler indépendamment aussi bien l'agent liant-désintégrant que les excipients usuels de formulation, notamment par granulation par voie humide et de les mélanger directement avec les particules enrobées de principe actif.

Toutefois, ce procédé est relativement lourd puisque nécessitant des étapes additionnelles et en préférera au contraire intégrer directement les excipients au principe actif enrobé et ce, avant l'étape ultérieure de compression ou extrusion.

La technique de "hot melt coating" et son matériel de mise en oeuvre sont connus et ont largement été décrits dans les documents ACHANTE AS et al, « Development of hot melt coatings methods » dans DRUG DEV IND PHARM, Volume 23, Numéro 5, 1997, pages 441 à 449, et JOZWIAKOWSKI MJ et al, « Characterization of hot melt fluid bed coating process for fine granule » dans PHARM RES, Volume 7, Numéro 11, 1990, pages 1119-1126.

Dans la suite de la description, on illustre le procédé de "hot melt coating" de l'invention par la technique du lit d'air fluidisé bien que d'autres techniques connues puissent être envisagées.

Selon une caractéristique avantageuse du procédé de l'invention, la température de chauffage de la poudre de principe actif est choisie dans une plage allant d'environ 1° C en dessous à 2° C au dessus de la fourchette du point de fusion de l'agent lipidique d'enrobage choisi déterminé lors de son analyse, soit par la méthode du tube capillaire, soit par la méthode du point de goutte.

De même, pour favoriser l'enrobage de l'agent lipidique sur les particules de principe actif, celui-ci est chauffé et maintenu à une température comprise entre son point de fusion et 50°C au dessus dudit point de fusion.

Pour une température inférieure au point de fusion, l'agent lipidique est solide et ne peut être pulvérisé.

Pour une température supérieure à 50° C au dessus du point de fusion, la solidification de l'agent d'enrobage est trop longue et provoque l'agglomération des particules.

Pour permettre d'obtenir un enrobage d'agent lipidique sous forme d'un film fin et homogène tout en évitant d'être brisé lors de l'étape de compression, le débit de pulvérisation d'agent lipidique est choisi en fonction de la masse de principe actif à enrober et de la taille du lit d'air fluidisé.

En pratique, le débit de pulvérisation d'agent lipidique est compris entre 5 et 500 grammes par minute.

En pratique, dans le cas de lot pilote dont la quantité va jusqu'à 10 kg d'actif à enrober, le débit de pulvérisation de l'agent lipidique est compris entre 5 et 20 grammes par minute, avantageusement entre 8 et 13 grammes par minute.

Concernant les lots industriels dont le poids en principe actif va jusqu'à 400 kg par lot, le débit de pulvérisation de l'agent lipidique est choisi entre 200 et 500 grammes par minute, avantageusement 280 à 360 grammes par minute.

Selon une autre caractéristique du procédé de l'invention, la pression d'atomisation correspond aux conditions dites "de pelliculage" et est supérieure à 2,5 bars.

Concernant les lots pilotes, elle est avantageusement choisie supérieure à 3 bars.

De même, concernant les lots industriels, les débits étant plus élevés, la pression d'atomisation est choisie supérieure à 4 bars, avantageusement entre 4 et 5 bars.

S'agissant du matériel, on met avantageusement en oeuvres des buses binaires d'atomisation d'air dont le nombre et l'ouverture sont adaptés en fonction du débit de pulvérisation et de la taille du lit d'air fluidisé.

Par ailleurs, selon une autre caractéristique de l'invention, la taille granulométrique du principe actif est comprise entre 5 et 500 micromètres, étant entendu que la majorité des particules se trouve dans une fourchette de 200 micromètres, de sorte à favoriser la mise en mouvement et l'obtention de surfaces particulaires homogènes dans le lit de fluidisation.

Selon un mode de réalisation préféré, l'agent lipidique est un ester de glycérol et d'un mélange d'acide stéarique et palmitique commercialisé par le Demandeur sous la marque PRECIROL® ATO 5 et correspondant à la monographie de la pharmacopée européenne 2000.1428.

Comme déjà dit, l'agent lipidique est enrobé à raison d'au plus 10 % en poids du poids du principe actif enrobé.

En d'autres termes et comme déjà dit, l'enrobage d'un principe actif par une fine pellicule d'un agent lipidique spécifique et l'association à un agent liant-désintégrant dans un comprimé à croquer, permet de libérer le principe actif rapidement, à savoir plus de 80 % en 30 minutes, et ce, tout en masquant le goût amer du principe actif.

Selon une autre caractéristique du procédé de l'invention, l'agent liant-désintégrant est choisi dans le groupe comprenant l'hydroxypropy éther de cellulose.
Avantageusement, hydroxypropyl éther de cellulose contient de 7 à 12,9 % d'unité hydroxy propoxyl.

Comme déjà dit, outre l'agent liant-désintégrant, les particules enrobées sont mélangées avec des excipients usuels dans la fabrication des comprimés à croquer choisis dans le groupe comprenant les agents diluants, les agents lubrifiants, les arômes, les édulcorants.

De plus, l'invention se rapporte au comprimé à croquer susceptible d'être obtenu selon le procédé ci-avant.

L'invention et les avantages qui en découlent ressortiront mieux des exemples de réalisation suivants à l'appui des figures annexées.

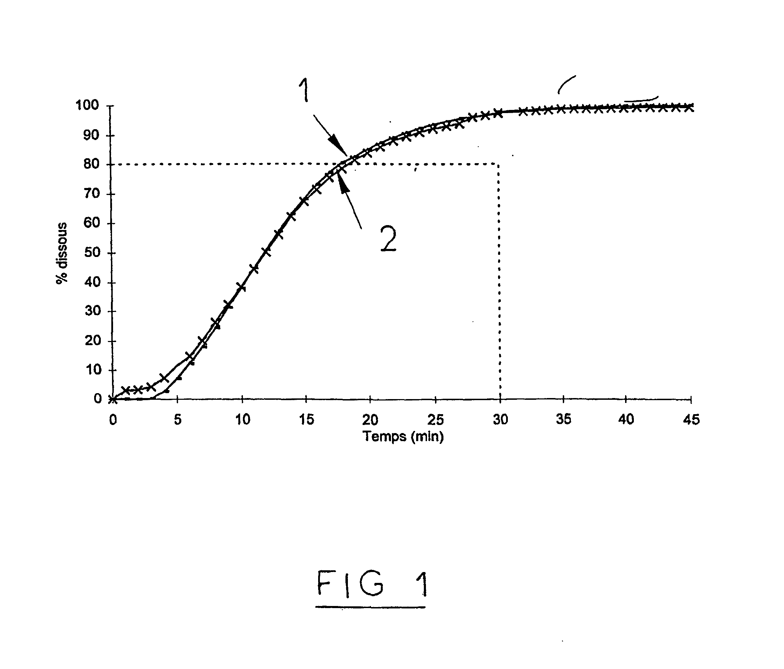

La figure 1 est une représentation du profil de dissolution d'un comprimé à croquer de paracétamol fabriqué selon le procédé de l'invention.

Dans l'exemple qui suit, on prépare deux lots d'un comprimé à croquer dosé à 80 mg de paracétamol, respectivement les lots numéros 1 et 2. La composition des comprimés constituant chacun de ces lots est représentée dans le tableau ci-après, dans lequel les pourcentages sont donnés en pourcentage du poids d'un comprimé.

| **Nom Commercial** | **Nom usuel** | **Fonction** | **Lot 1** | **Lot 2** |
|---|---|---|---|---|
| GATTAPHEN ® T | Paracétamol enrobé de 5% de PRECIROL® | Principe actif | 21,5 | 21,5 |
| EMDEX ® | Dextrate | diluant co-édulcorant | 33,4 | 33,4 |
| PEARLITOL ® 400 DC | Mannitol | diluant co-édulcorant | 32,2 | 32,2 |
| NEOSORB ® P20/60 | Sorbitol | diluant co-édulcorant | 3,0 | 3,0 |
| XYLISORB ® | Xylitol | diluant co-édulcorant | 3,0 | 3,0 |
| LH 21 | hydroxypropyl cellulose faiblement substitué | liant-désintégrant | 3,5 | 3,5 |
| ACDISOL ® | CMC Na | désintégrant | 1,5 | 1,5 |
| NUTRASWEET ® | Aspartame | édulcorant | 0,7 | 0,7 |
| SUNETT ® | K acesulfame | édulcorant | 0,3 | 0,3 |
| Grape CK | | arôme | - | 0,2 |
| Vanilla IFF | | arôme | - | 0,02 |
| Chroma-tone DDB 8297-P colouring | | colorant | - | 0,2 |
| Magnésium stéarate | | lubrifiant | 0,7 | 0,7 |

GATTAPHEN ® T commercialisé par GATTEFOSSE
EMDEX® commercialisé par MENDELL
PEARLITOL®, NEOSORB® et XYLISORB® fournis par la société ROQUETTE Frères
LH 21 fourni par SHINETSU
ACDISOL® fourni par la société SEPPIC
NUTRASWEET® fourni par la société ARNAUD Etablissement
SUNETT® fourni par la société NUTRINOVA France
Arôme Vanille 22b249 fourni par la société IFF
Arôme GRAPE FL SD ART D 1439500 et Colorant CHROMA-TONE DDB 8297-P fournis par la société CROMPTON & KNOWLES

Pour la fabrication des comprimés, on procède comme suit.

On chauffe la poudre de principe dont la taille granulométrique est comprise entre 50 et 500 micromètres jusqu'à une température de fusion de l'agent lipidique en l'espèce aux alentours de 53° C au moyen d'un lit d'air fluidisé de sorte à obtenir des particules individualisées.

On liquéfie séparément l'agent lipidique constitué d'un ester de glycérol et d'un mélange d'acide palmitique et d'acide stéarique commercialisé par le Demandeur sous la marque PRECIROL ® ATO 5 à 110° C.

On pulvérise ensuite l'agent d'enrobage lipidique sur la poudre de principe actif chauffée afin de permettre la répartition homogène de l'agent matriciel lipidique autour des particules de paracétamol. Lors de cette étape d'enrobage, la pression d'air d'atomisation est fixée à au moins 2,5 bars pour un débit de pulvérisation de PRECIROL® de 8 grammes par minute.

Les particules enrobées ainsi obtenues sont ensuite refroidies jusqu'à température ambiante et correspondent au produit commercialisé par le Demandeur sous la marque GATTAPHEN ®. Les particules sont ensuite mélangées à température ambiante avec l'ensemble des excipients figurant dans le tableau ci-dessus.

On soumet enfin le mélange obtenu à une étape de compression usuelle de sorte à obtenir les comprimés à croquer de l'invention. La machine mise en oeuvre est une comprimeuse rotative du type MANESTY, KILLIAN ou KORCH.

Comme déjà dit, est représenté sur la figure 1 le profil de dissolution des deux lots de comprimés fabriqués (le lot 1 correspondant à la courbe 1, le lot 2 correspondant à la courbe 2). Comme le montrent ces courbes, on obtient une libération du paracétamol, qui doit être considérée comme immédiate dans la mesure où 80 % du principe actif est libéré en 30 minutes, et ce, conformément aux dispositions de la pharmacopée américaine (USP XXIII).

L'invention et les avantages qui en découlent ressortent bien de la description qui précède.

On notera plus particulièrement la composition et l'originalité du procédé de fabrication en intégrant des particules de principe actif enrobées d'un agent lipidique, donc sans solvant, et ce, dans des proportions d'au plus 10 % de lipide spécifique, tendant donc à diminuer considérablement le coût de production du comprimé. De même, la formation d'un film homogène et fin autour des particules de principe actif:
◆ d'une part, permet d'obtenir la libération immédiate du principe actif et ce conjugué avec l'effet désintégrant de l'agent liant-désintégrant ;
◆ et d'autre part, permet d'éviter d'enrober partiellement les grains de principe actif et donc la diffusion immédiate du goût amer du principe actif.

De la sorte, le goût du principe actif est complètement masqué et les comprimés à croquer de par leur composition présentent une palatabilité et un croquage satisfaisant.

En outre et surtout, on obtient grâce à l'incorporation d'un agent liant-désintégrant une libération immédiate du principe actif par désagrégation du comprimé et dissolution sans effet prolongé de l'actif enrobé.

## Revendications

1. Comprimé à croquer à goût masqué et à libération immédiate du principe actif **caractérisé en ce qu'**il est constitué d'un mélange comprenant :
• des particules individualisées de principe actif enrobés d'un agent lipidique obtenu par réaction entre au moins un polyhydroxyalcool et au moins un acide gras saturé libre ou estérifié, ledit acide gras comprenant de 8 à 22 atomes de carbone ;
• au moins un agent liant-désintégrant choisi dans le groupe consistant en hydroxypropyl éther de cellulose faiblement substitué, dans lequel une faible proportion des trois groupes hydroxyle contenus dans la chaîne β-o-glucopyranosyl de la cellulose sont estérifiés par de l'oxyde de propylène ;
• au moins un excipient choisi dans le groupe comprenant les arômes et les édulcorants.

2. Comprimé à croquer selon la revendication 1, **caractérisé en ce que** le point de fusion de l'agent lipidique est compris entre 37 et 75° C.

3. Comprimé selon la revendication 3, **caractérisé en ce que** l'agent lipidique est un ester de glycérol et d'un mélange d'acide stéarique et d'acide palmitique.

4. Comprimé selon l'une des revendications 1 à 3, **caractérisé en ce que** l'agent lipidique représente au plus 10 % en poids du poids de principe actif.

5. Comprimé selon la revendication 4, **caractérisé en ce que** l'agent lipidique représente de 2 à 5 % du poids de principe actif.

6. Comprimé selon la revendication 1, **caractérisé en ce que** l'hydroxypropyl éther de cellulose faiblement substitué contient de 7 à 16 % d'unité hydroxypropoxyl.

7. Comprimé selon la revendication 6, **caractérisé en ce que** l'hydroxypropyl éther de cellulose faiblement substitué contient de 7 à 12,9% d'unité hydroxypropoxyl.

8. Comprimé selon l'une des revendications précédentes, **caractérisé en ce que** l'agent liant - désintégrant représente entre 0,1 et 5 % en poids du poids d'un comprimé.

9. Procédé de fabrication de comprimés à croquer à goût masqué et libération immédiate de principe actif comprenant les étapes suivantes respectivement:
• une étape d'enrobage lors de laquelle :
· tout d'abord, on chauffe tout en fluidisant, une poudre constituée de particules individualisées de principe actif à une température voisine du point de fusion de l'agent lipidique mis en oeuvre ultérieurement ;
· on pulvérise ensuite, sur les particules individualisés en l'absence de tout solvant, toujours à chaud, à l'état fondu, un agent lipidique obtenu par réaction entre au moins un polyhydroxyalcool et au moins un acide gras saturé libre ou estérifié, ledit acide gras comprenant de 8 à 22 atomes de carbone ;
· on refroidit les particules enrobées jusqu'à température ambiante;
• une étape de mise en forme lors de laquelle :
· on mélange préalablement à température ambiante, les particules ainsi enrobées avec au moins un agent liant-désintégrant choisi dans le groupe consistant en hydroxypropyl éther de cellulose faiblement substitué, dans lequel une faible proportion des trois groupes hydroxyle contenus dans la chaîne β-o-glucopyranosyl de la cellulose sont estérifiés par de l'oxyde de propylène, et au moins un excipient choisi dans le groupe comprenant les arômes et les édulcorants ;
· puis, on soumet le mélange obtenu à l'étape de mise en forme proprement dite afin d'obtenir des comprimés.

10. Procédé de fabrication selon la revendication 9, **caractérisé en ce que** l'étape de mise en forme consiste en une compression.

11. Procédé de fabrication selon la revendication 9, **caractérisé en ce que** l'étape de mise ne forme consiste en une extrusion.

12. Procédé de fabrication selon l'une des revendications 9 à 11, **caractérisé en ce que** la température de chauffage de la poudre de principe actif est choisie dans une plage allant d'environ 1° C en dessous à 2° C au dessus de la fourchette du point de fusion de l'agent lipidique d'enrobage.

13. Procédé selon l'une des revendications 9 à 12, **caractérisé en ce que** l'agent lipidique est fondu et maintenu à une température comprise entre son point de fusion et 50°C au dessus dudit point de fusion.

14. Procédé selon l'une des revendications 9 à 13, **caractérisé en ce que** le débit de pulvérisation d'agent lipidique est compris entre 5 et 500 grammes par minute.

15. Procédé selon l'une des revendications 10 à 14, **caractérisé en ce que** la pression d'air d'atomisation est supérieure à 2,5 bars.

16. Procédé selon l'une des revendications 9 à 15, **caractérisé en ce que** l'agent lipidique est un ester de glycérol et d'un mélange d'acide stéarique et d'acide palmitique.

17. Procédé selon l'une des revendications 9 à 16, **caractérisé en ce que** l'agent lipidique est pulvérisé à raison d'au plus 10 % en poids du poids de principe actif.

18. Procédé selon la revendication 17, **caractérisé en ce que** l'agent lipidique est pulvérisé à raison de 2 à 5% en poids du poids de principe actif.

19. Procédé selon la revendication 9, **caractérisé en ce que** l'hydroxypropyl éther de cellulose faiblement substitué contient de 7 à 16% d'unité hydroxypropoxyl.

20. Procédé selon la revendication 19, **caractérisé en ce que** l'hydroxypropyl éther de cellulose faiblement substitué contient de 7 à 12,9% d'unité hydroxypropoxyl.

21. Procédé selon l'une des revendications 9 à 20, **caractérisé en ce que** l'agent liant-désintégrant représente entre 0,1 et 5 % en poids du poids d'un comprimé.

22. Comprimé à croquer susceptible d'être obtenu par le procédé selon l'une des revendications 9 à 21.

## Claims

1. Taste-masked chewable tablet with immediate release of the active principle, **characterized in that** it consists of a mixture comprising:
• individualized active principle particles coated with a lipid agent obtained by reaction between at least one polyhydroxy alcohol and at least one free or esterified saturated fatty acid, said fatty acid comprising from 8 to 22 carbon atoms;
• at least one disintegrating binder consisting of a cellulose hydroxypropyl ether in which a low proportion of the three hydroxyl groups contained in the β-O-glucopyranosyl chain of the cellulose are esterified with propylene oxide;
• at least one excipient chosen from the group comprising flavourings and sweeteners.

2. Chewable tablet according to Claim 1, **characterized in that** the melting point of the lipid agent is between 37 and 75°C.

3. Tablet according to Claim 1, **characterized in that** the lipid agent is an ester of glycerol and of a mixture of stearic acid and palmitic acid..

4. Tablet according to one of Claims 1 to 3, **characterized in that** the lipid agent represents not more than 10% by weight of the weight of active principle.

5. Tablet according to Claim 4, **characterized in that** the lipid agent represents from 2 to 5% of the weight of active principle.

6. Tablet according to Claim 1, **characterized in that** the weakly substituted cellulose hydroxypropyl ether contains from 7 to 16% of hydroxypropyl units.

7. Tablet according to Claim 6, **characterized in that** the weakly substituted cellulose hydroxypropyl ether contains from 7 to 12.9% of hydroxypropyl units.

8. Tablet according to one of the preceding claims, **characterized in that** the disintegrating binder represents between 0.1 and 5% by weight of the weight of a tablet.

9. Process for manufacturing taste-masked chewable tablets with immediate release of active principle, comprising the following steps, respectively:
• a coating step during which:
· firstly, a powder consisting of individualized active principle particles is heated, while at the same time fluidizing, to a temperature close to the melting point of the lipid agent used later;
· a lipid agent obtained by reaction between at least one polyhydroxy alcohol and at least one free or esterified saturated fatty acid, said fatty acid comprising from 8 to 22 carbon atoms, is then sprayed onto the individualized particles in the absence of any solvent, while still under hot conditions, in the molten state;
· the coated particles are cooled to room temperature;
• a shaping step during which:
· the particles thus coated are premixed, at room temperature, with at least one disintegrating binder chosen from the group consisting in a cellulose hydroxypropyl ether in which a low proportion of the three hydroxyl groups contained in the β-O-glucopyranosyl chain of the cellulose are esterified with propylene oxide, and at least an excipient chosen from the group comprising flavourings and sweeteners;
· the mixture obtained is then subjected to the actual shaping step in order to obtain tablets.

10. Manufacturing process according to Claim 9, **characterized in that** the shaping step consists of a tabletting operation.

11. Manufacturing process according to Claim 9, **characterized in that** the shaping step consists of an extrusion operation.

12. Manufacturing process according to one of Claims 9 to 11, **characterized in that** the heating temperature of the active principle powder is chosen within a range from about 1°C below to 2°C above the melting point range of the coating lipid agent.

13. Process according to one of Claims 9 to 12, **characterized in that** the lipid agent is melted and kept at a temperature between its melting point and 50°C above said melting point.

14. Process according to one of Claims 9 to 13, **characterized in that** the flow rate of spraying of lipid agent is between 5 and 500 grams per minute.

15. Process according to one of Claims 10 to 14, **characterized in that** the spraying air pressure is greater than 2.5 bar.

16. Process according to one of Claims 9 to 15, **characterized in that** the lipid agent is an ester of glycerol and of a mixture of stearic acid and palmitic acid.

17. Process according to one of Claims 9 to 16, **characterized in that** the lipid agent is sprayed in a proportion of not more than 10% by weight of the weight of active principle.

18. Process according to Claim 17, **characterized in that** the lipid agent is sprayed in a proportion of from 2 to 5% by weight of the weight of active principle.

19. Process according to Claim 9, **characterized in that** the weakly substituted cellulose hydroxypropyl ether contains from 7 to 16% of hydroxypropyl units.

20. Process according to Claim 19, **characterized in that** the weakly substituted cellulose hydroxypropyl ether contains from 7 to 12.9% of hydroxypropyl units.

21. Process according to one of Claims 9 to 20, **characterized in that** the disintegrating binder represents between 0.1 and 5% by weight of the weight of a tablet.

22. Chewable tablet obtainable by the process according to one of Claims 9 to 21.

## Patentansprüche

1. Geschmacksmaskierte Kautablette mit sofortiger Wirkstoff-Freisetzung, **dadurch gekennzeichnet, daß** sie aus einer Mischung besteht, welche folgende Bestandteile enthält:
• individualisierte Partikel des Wirkstoffs, welche mit einer Lipidsubstanz ummantelt sind, die durch Reaktion zwischen mindestens einem Polyhydroxyalkohol und mindestens einer freien oder veresterten gesättigten Fettsäure erhalten sind, wobei die genannte Fettsäure zwischen 8 und 22 Kohlenstoffatome aufweist;
• mindestens ein auflösbares Bindemittel, ausgewählt aus der aus leicht substituierten Cellulose-Hydroxypropylether bestehenden Gruppe, bei dem ein geringer Anteil der drei in der β-o-Glucopyranosyl-Kette der Cellulose enthaltenen Hydroxyl-Gruppen mit Propylenoxid verestert ist;
• mindestens einen Hilfsstoff, der aus der Aromen und Süßungsmittel enthaltenden Gruppe ausgewählt ist.

2. Kautablette nach Anspruch 1, **dadurch gekennzeichnet, daß** der Schmelzpunkt der Lipidsubstanz zwischen 37 und 75 °C liegt.

3. Tablette nach Anspruch 1, **dadurch gekennzeichnet, daß** die Lipidsubstanz ein Ester aus Glycerin und einer Mischung von Stearinsäure und Palmitinsäure ist.

4. Tablette nach einem der Ansprüche 1 bis 3, **dadurch gekennzeichnet, daß** die Lipidsubstanz höchtens 10 Gewichtsprozent des Gewichts des Wirkstoffs darstellt.

5. Tablette nach Anspruch 4, **dadurch gekennzeichnet, daß** die Lipidsubstanz 2 bis 5% des Gewichts des Wirkstoffs darstellt.

6. Tablette nach Anspruch 1, **dadurch gekennzeichnet, daß** der leicht substituierte Cellulose-Hydroxypropylether 7 bis 16% der Hydroxypropoxyleinheit enthält.

7. Tablette nach Anspruch 6, **dadurch gekennzeichnet, daß** der leicht substituierte Cellulose-Hydroxypropylether 7 bis 12,9% der Hydroxypropoxyl-einheit enthält.

8. Tablette nach einem der vorangehenden Ansprüche, **dadurch gekennzeichnet, daß** das auflösbare Bindemittel zwischen 0,1 und 5% Gewichtsprozent des Gewichts einer Tablette darstellt.

9. Verfahren zur Herstellung geschmacksmaskierter Kautabletten mit sofortiger Wirkstoff-Freisetzung, welches die folgenden Schritte enthält:
• ein Schritt der Ummantelung, bei dem:
· zunächst ein aus individualisierten Partikeln des Wirkstoffs bestehendes Pulver auf eine Temperatur nahe dem Schmelzpunkt der später verwendeten Lipidsubstanz unter Fluidisierung erhitzt wird;
· anschließend auf die individualisierten Partikel in Abwesenheit jeglichen Lösungsmittels nach wie vor im erhitzten Zustand eine Lipidsubstanz in geschmolzenem Zustand aufgesprüht wird, welche durch Reaktion zwischen mindestens einem Polyhydroxylalkohol und mindestens einer freien oder veresterten, gesättigten Fettsäure erhalten wurde, wobei die genannte Fettsäure zwischen 8 und 22 Kohlenstoffatome aufweist;
· Abkühlen der ummantelten Partikel bis auf Umgebungstemperatur;
• ein Formungsschritt, bei dem:
· zuerst bei Umgebungstemperatur die so ummantelten Partikel mit mindestens einem auflösbaren Bindemittel, ausgewählt aus der aus leicht substituierten Cellulose-Hydroxypropylether bestehenden Gruppe, bei dem ein geringer Anteil der drei in der β-o-Glucopyranosyl-Kette der Cellulose enthaltenen Hydroxyl-Gruppen mit Propylenoxid verestert ist, und mit mindestens einem Hilfsstoff, der aus der Aromen und Süßungsmittel enthaltenden Gruppe ausgewählt ist, gemischt werden;
· anschließend die erhaltene Mischung einem eigentlichen Formungsschritt unterzogen wird, um Tabletten zu erhalten.

10. Herstellungsverfahren nach Anspruch 9, **dadurch gekennzeichnet, daß** der Formungsschritt aus einem Pressen besteht.

11. Herstellungsverfahren nach Anspruch 9, **dadurch gekennzeichnet, daß** der Formungsschritt aus einer Extrusion besteht.

12. Herstellungsverfahren nach einem der Ansprüche 9 bis 11, **dadurch gekennzeichnet, daß** die Temperatur der Erhitzung des Wirkstoffpulvers in einem Bereich, der von etwa 1°C unterhalb bis 2°C oberhalb der Spanne des Schmelzpunktes der Lipidsubstanz zur Ummantelung reicht, gewählt wird.

13. Verfahren nach einem der Ansprüche 9 bis 12, **dadurch gekennzeichnet, daß** die Lipidsubstanz geschmolzen und auf einer Temperatur gehalten wird, die zwischen ihrem Schmelzpunkt und 50°C oberhalb des genannten Schmelzpunktes liegt.

14. Verfahren nach einem der Ansprüche 9 bis 13, **dadurch gekennzeichnet, daß** der Sprühdurchsatz der Lipidsubstanz zwischen 5 und 500 Gramm pro Minute liegt.

15. Verfahren nach einem der Ansprüche 10 bis 14, **dadurch gekennzeichnet, daß** der Luftdruck zur Zerstäubung oberhalb von 2,5 bar liegt.

16. Verfahren nach einem der Ansprüche 9 bis 15, **dadurch gekennzeichnet, daß** die Lipidsubstanz ein Glycerolester ist und aus einer Mischung aus Stearinsäure und Palmitinsäure besteht.

17. Verfahren nach einem der Ansprüche 9 bis 16, **dadurch gekennzeichnet, daß** die Lipidsubstanz im Verhältnis von höchstens 10 Gewichtsprozent des Gewichts des Wirkstoffs versprüht wird.

18. Verfahren nach Anspruch 17, **dadurch gekennzeichnet, daß** die Lipidsubstanz im Verhältnis von 2 bis 5 Gewichtsprozent des Gewichts des Wirkstoffs versprüht wird.

19. Verfahren nach Anspruch 9, **dadurch gekennzeichnet, daß** der leichtsubstituierte Cellulose-Hydroxypropylether 7 bis 16% der Hydroxypropoxyl-Einheit enthält.

20. Verfahren nach Anspruch 19, **dadurch gekennzeichnet, daß** der leichtsubstituierte Cellulose-Hydroxypropylether 7 bis 12,9 % der Hydroxypropoxyl-Einheit enthält.

21. Verfahren nach einem der Ansprüche 9 bis 20, **dadurch gekennzeichnet, daß** das auflösbare Bindemittel zwischen 0,1 und 5 Gewichtsprozent des Gewichts einer Tablette ausmacht.

22. Kautablette, die dazu geeignet ist, nach dem Verfahren gemäß einem der Ansprüche 9 bis 21 erhalten zu werden.
